# EUROPEAN PATENT APPLICATION

(11) **EP 3 705 170 A1**
(43) Date of publication of application: **09.09.2020**
(21) Application number: 19161562.4
(22) Date of filing: 08.03.2019
(51) Int. Cl.: B01D 53/047, B01D 53/22, C10L 3/10, C12M 1/107

(54) **BIOGAS PLANT AND BIOGAS TREATMENT**

(71) Applicant: Hitachi Zosen Inova AG, 8005 Zürich (CH)
(72) Inventor: Becker, Jens, 8005 Zurich (CH); Wiegers, Jan, 8005 Zurich (CH)
(74) Representative: Schaad, Balass, Menzl & Partner AG

(57) **Abstract**

The present invention relates to a biogas plant (10) including a fermenter (12), a biogas processing unit (16) and a thermal engine (14). The biogas processing unit (16) includes a gas separation unit (32) and a compression unit (33) for the separation of raw biogas (26) from the fermenter (12) into two gas streams, whereby the first gas stream (27) includes a product gas (28) that is enriched in biomethane (17) with respect to the composition of the raw biogas (26) and the second gas stream includes a residual gas (30) that is enriched with carbon dioxide with respect to the composition of the raw biogas (26). At least a fraction of the residual gas (30) is fed to the thermal engine (14), where energy (39) is produced that is used for operation of the compression unit (33).

## Description

The present invention relates to a biogas plant with a fermenter and a biogas treatment plant according to claim 1, and to a method for obtaining biomethane from raw biogas according to claim 14.

The term biogas usually refers to a mixture of mostly methane and carbon dioxide, which are formed through fermentation of organic substances in the absence of oxygen. Also the gas, which is liberated from landfills or digestion towers on sewage treatment plants may be called biogas. A biogas plant is therefore a plant for the production of biogas from organic substrates. Originally, biogas has been used in local cogeneration units to produce electric energy and heat, while parts of the generated heat could further be used for the biogas production process.

If biogas is needed at a location remote from its origin, processing of the biogas to biomethane and feeding thereof into a gas grid or a storage facility is necessary. For this purpose, biogas treatment plants have been developed, which are used for the separation of biogas into its components, mainly biomethane and carbon dioxide. The aim is to obtain an essentially pure methane gas stream, which is subsequently fed into a gas grid or can be used e.g. in the mobility industry.

Several methods for the purification of raw biogas to obtain biomethane have been successfully used in practice. Known methods include water scrubbing, organic scrubs (e.g. with amine-based solvents), pressure swing adsorption (PSA) and membrane based gas permeation methods.

Pressure swing adsorption methods include feeding of the biogas under elevated pressure into a solid-state bed reactor that is filled with an adsorbent. The so-called "heavy components" of the biogas mixture are adsorbed by the adsorbent and the so-called "light components" can be retrieved at the outlet of the bed in concentrated form. If the adsorption bed is saturated, part of the heavy components will also escape from the bed. As soon as this happens, the process is switched using valves, such that the outlet intended for the light components gets closed and a separate second outlet for the heavy components is opened. This switch comes along with a pressure reduction. At this reduced pressure the heavy absorbed components desorb from the bed and can be retrieved at the second outlet. Two interchangeably loaded and de-loaded adsorbents allow a continuous operation. If pressures below atmospheric pressure are used, the method is also termed VSA (Vacuum Swing Adsorption). If the pressure is partly above atmospheric pressure and partly below, then the method is termed VPSA (Vacuum Pressure Swing Adsorption). With the exception of the applied pressures and the necessary precautions associated therewith, all these methods are basically identical.

Membrane-based gas permeation methods include separation of carbon dioxide from the biogas with the aid of membranes. For that purpose, the raw biogas is pre-cleaned and compressed to pressures of usually 10 to 30 bar. At this pressure, carbon dioxide can easily permeate through a membrane that separates a permeate side from a retentate side. Other components of the raw biogas, in particular biomethane, cannot that easily permeate through the membrane, such that carbon dioxide ("residual gas") can be collected on the permeate side (i.e. as "permeate"), while the desired cleaned biomethane is provided as "product gas" on the retentate side (i.e. as "retentate"), under the pressure of the original compression and ready for being fed into a gas grid. If multilayer membranes are used, they usually comprise a solvent-selective pore-free polymer film, a microporous carrier membrane and a permeable fleece. Inorganic gas separation membranes e.g. based on zeolites, are available as alternative to polymer film based membranes. Irrespective of the membrane type multistep membrane systems are often used in order to obtain gas streams of biomethane and carbon dioxide with sufficiently high purity.

WO 2012/000727 A1 discloses a three-step process and an apparatus for separation of gas mixtures. The process and the apparatus consist of a feed stream separation stage and a retentate separation stage, of which both are membrane separation stages. The first retentate stream is heated to a temperature higher than the temperature of the feed stream, before it is introduced to the retentate separation stage, and the total capacity of the membranes used in the retentate separation stage is higher than the total capacity of the membranes used in the feed stream separation stage. The process and the apparatus are able to afford both permeate gas and retentate gases in relatively high purities with low re-circulation flow. This method is often used in practice and has been licensed to many suppliers in the past.

One problem of current biogas treatment methods is that they require a high amount of energy. For instance, the above-mentioned scrubbing methods require a lot of thermal energy, while the pressure swing adsorption methods and the membrane-based separation methods have a high energy demand for the compression of the raw biogas. Until now, the required compression energy has usually been exclusively provided by electric engines, which causes high production costs. Because of the continuously increasing demands with respect to purity of the biomethane and CO₂ - and maximum tolerable amounts of pollutants therein -, multistep separation and cleaning systems are usually necessary. However, each cleaning step increases the overall energy consumption of the cleaning process.

Apart from the desired product gas of more or less pure biomethane, raw biogas also contains a variable amount of carbon dioxide that is obtained as a side product (residual gas). So far, this gas stream of carbon dioxide was considered to be of little value and has usually been released into the environment. In view of the climate change, new ways to reduce the CO₂ emissions have come into focus. One example is the use of said biomass derived CO₂ as raw material for production or energy conversion processes.

The problem to be solved by the present invention is therefore to provide a biogas plant comprising a biogas production facility for the production of biogas and a biogas treatment plant for the separation of raw biogas into biomethane and carbon dioxide, whereby the biogas plant has an improved energy and economical balance and at the same time meets the requirements with respect to purity of the gas streams obtained from the separation of the raw biogas.

This problem is solved by a biogas plant according to claim 1 and a method with the features of claim 14. Preferred embodiments are subject of the dependent claims.

A biogas plant in accordance with the present invention includes
- a fermenter (or "digester") for the production of raw biogas from organic biomaterial (or "biomass"), in particular organic waste,
- a biogas treatment plant, and
- a thermal engine.

The fermenter is capable of converting biomass into raw biogas and generally contains microorganisms that are capable of digesting the organic biomaterial. The fermenter can be an assigned technical unit (e.g. an apparatus) or a facility (e.g. a waste disposal site). The digestion - or "fermentation" - of the biomaterial leads to the production of raw biogas that contains biomethane.

To separate the biomethane in the raw biogas produced by the fermenter from other biogas components, in particular carbon dioxide (CO₂), the biogas treatment plant includes a gas separation unit that divides the raw biogas into at least a first and a second gas stream. The first gas stream consists of a product gas that is enriched in biomethane with respect to the composition of the raw biogas and the second gas stream consists of a residual gas that is enriched in carbon dioxide with respect to the composition of the raw biogas. The biogas treatment plant can comprise more than one separation step. Therefore the at least one residual gas stream can be a mixture of different intermediate residual gas streams gained from the different separation steps.

The separation of the gas streams can be achieved by using one of the known methods mentioned in the introduction part of this application, in particular by membrane separation or pressure swing adsorption. Since these separation methods generally require compression of the raw biogas to be separated, the biogas treatment plant includes a compression unit that can perform this task.

The thermal engine is part of the biogas plant and includes a combustion unit that is provided with a combustion gas for combustion, which produces thermal energy. The combustion unit can for example be a gas piston engine or a micro gas turbine. In addition to the combustion unit the thermal engine further includes an exhaust gas treatment unit. Said exhaust gas treatment unit is provided with an exhaust gas for treatment, which generally also produces thermal energy. The thermal engine preferably also comprises at least one heat exchanger to utilize said thermal energy.

A key aspect of the present invention is
- that at least a fraction of the residual gas is fed to the thermal engine and delivered to the combustion unit as part of the combustion gas and/or to the gas treatment unit as part of the exhaust gas, and
- that energy produced by combustion of the combustion gas and/or treatment of the exhaust gas is used for operating the compression unit.

In other words, in accordance with the present invention, at least a fraction of the residual gas (obtained from the separation of the raw biogas within the gas separation unit) is delivered to one or both of the combustion unit and the exhaust gas treatment unit. Of said fraction of residual gas fed to the thermal engine a fraction X (with X being between 0% and 100%) is delivered to the combustion unit and forms part of the combustion gas and the remaining fraction 100%-X is delivered to the exhaust gas treatment unit and forms part of the exhaust gas.

Preferably, the exhaust gas includes exhaust gases from the combustion process within the combustion unit. This way, the exhaust gases produced during combustion of the combustion gas can be cleaned, in particular by removal of acidic substances and/or nitrous oxides. Any residual gas delivered to the exhaust gas treatment unit is preferably mixed with the exhaust gases form the combustion unit before being fed into the exhaust gas treatment unit.

As mentioned, the combustion of the combustion gas and generally also the treatment of the exhaust gas produces energy can be used for the operation of the compression unit of the gas separation unit. The energy transfer from the thermal engine to the compression unit can be either direct (e.g. via a rigid or variable coupling) or indirect with an energy converter or storage in between. In consequence, operation of the compression unit requires less externally produced energy (i.e. electric energy or fuel produced outside of the biogas plant), which can reduce the overall production costs.

Also, the biogas plant according to the present invention allows for an improved environmental balance, since any methane residue present in the residual gas can be converted into carbon dioxide and water during its combustion in the thermal engine and is therefore not released as a potent greenhouse gas into the environment. Normally, the goal of a gas separation unit is to minimize the amount of residual methane within the residual gas, i.e. the methane slip. A reduction in methane slip usually goes along with a more complex construction of the gas separation unit (e.g. increased number of separation stages, larger membrane surface area etc.). However, since the biogas plant according to the present invention allows for using the residual methane within the residual gas for energy production (and/or at least allows for removing it with the aid of the exhaust gas treatment unit, e.g. by means of oxidation), the gas separation unit of the inventive biogas plant can be significantly simplified.

In summary, thanks to reduced need for external energy and significant reduction of the amount of methane that is liberated into the atmosphere, the biogas plant in accordance with the present invention provides a particularly energy-efficient, economically and environmentally friendly production of biomethane. Since a simplified separation unit can be used, the building and maintenance costs as well as the operating costs of the biogas plant can be substantially reduced.

Notably, any residual gas that is not fed to the thermal engine can be used for other purposes or it can be cleaned in conventional ways before being released into the atmosphere.

In a preferred embodiment of the biogas plant, the combustion gas includes a variable amount of natural gas and/or raw biogas from the fermenter and/or product gas. This means that in this preferred embodiment, the combustion gas may consist of only residual gas or it may additionally include fractions of the named combustible gases. The addition of combustible gases to the combustion gas can be required, because - depending on the separation efficiency of the gas separation unit (i.e. the methane slip of the gas separation unit) - the residual fraction of biomethane in the residual gas may be very small, such that the latter may not contain sufficient combustible components any more to allow adequate combustion within the combustion unit for energy production. In this case, a variable amount of natural gas and/or raw biogas and/or product gas can be added to the combustion gas to drive the combustion process. If applicable, also other combustibles, e.g. LPG (propane/butane), can be added to the combustion gas. This ensures that also in case of low biomethane concentrations in the residual gas, a constant operation of the combustion unit and therefore a constant energy production can be guaranteed. The minimal concentration of combustible gases within the combustion gas is determined by the type of thermal engine used. Pilot injection engines are one example of engines that allow combustion of lean gases.

In case that the thermal engine produces energy than cannot be utilized by the compression unit at a certain point in time, the extra energy is preferably fed to and utilized by other units of the biogas plant, e.g. the fermenter. Alternatively, such extra energy may also be fed into an energy storage or local energy distribution grid.

Preferably, the residual gas includes a methane concentration of at least 0.25%, more preferably of at least 1.5% and particularly preferably of at least 3%. Preferably, the combustion gas fed to the combustion unit comprises exclusively gases of the biogas plant itself. This means that in this embodiment, no external combustion gas (e.g. from a local natural gas grid) is required and the biogas plant can therefore be operated autonomously and location-independent. If no gas grid connection is present, the produced biomethane can be used immediately or filled into containers and transported in liquid or gaseous form, either to a consumer or to a facility, where it can be fed into a natural gas grid.

As mentioned, in addition to the combustion unit, the thermal engine further includes an exhaust gas treatment unit. Said exhaust gas treatment unit is preferably a catalytic gas processing unit and can be used for cleaning any part of residual gas that is not fed to the combustion unit. It is preferably also be used to clean the exhaust gases from the combustion unit before releasing them into the atmosphere. Methods for removing harmful substances, in particular nitrogen oxides, unburnt hydrocarbons and carbon monoxides are known to a person skilled in the art. For the removal of hydrocarbons and carbon monoxides common oxidation catalysts are often used (See e.g. J.K.Lampert et al, Applied Catalysis B: Environmental, Vol. 14, Issues 3-4, 29-12-1997, Pages 211-223).

The thermal engine, in particular the combustion unit, and the compression unit are preferably mechanically connected via a rigid coupling, or via a variable coupling. A variable coupling allows independent operation of the thermal engine and the compression unit. An example of a variable coupling is an intermediate torque converter, optionally with a bridging clutch.

The gas separation unit of the biogas plant is preferably a membrane separation plant and/or a pressure swing adsorption plant. In comparison to other methods, gas separation by means of a membrane separation plant or a pressure swing absorption plant is more energy-efficient and cost saving.

As mentioned, one advantage of the inventive biogas plant is that it can include a gas separation unit of simplified construction. "Simplified" means that in comparison to the common separation units, e.g. common membrane separation plants, the inventive biogas plant can have a reduced number of separation steps, a reduced number of membrane modules and/or an overall reduced size of membrane area. This reduction in separation steps or number or size of membrane module comes along with a higher methane slip. But as described above, the residual methane fraction within the residual gas can be utilized by the thermal engine for energy production. In terms of numbers this means that, instead of the nowadays common 0.5% methane slip (or even 0.25%), the residual gas may have a residual methane concentration of at least 1.5%, more preferably at least 3%.

In a specific embodiment, the separation unit is preferably a simplified membrane separation plant with two separation steps at most, particularly preferably with only one separation step. Compared to units having more than two separation steps, the inventive membrane separation unit requires substantially less energy, since each additional separation step increases the energy demand of the separation unit. In addition, the construction of a gas separation unit having only two or fewer separation steps is usually simpler and therefore cost saving and less demanding with respect to maintenance.

If a common gas (piston) motor is used as combustion unit, the combustion gas generally contains about 40% combustible material, usually biomethane. As mentioned, special lean gas motors are available, which are usually more expensive but can work with much lower methane concentrations in the combustion gas. From an economic point of view, the combustion gas preferably includes a biomethane concentration of at least 10%, more preferably at least 20%, even more preferably at least 30%.

Irrespective of the biomethane concentration within the residual gas it is preferred that all residual gas obtained in the gas separation unit is delivered directly or indirectly (e.g. via the fermenter or intermediate storage) to the thermal engine, preferably without prior treatment (treatment hereby refers to processes that change the composition of the residual gas). In the thermal engine, the residual gas is delivered to the combustion unit and/or the exhaust gas treatment unit. In one embodiment, all residual gas is fed to the combustion unit. Direct utilization of the residual gas saves costs and is thus particularly advantageous from an economic point of view.

In accordance with a preferred embodiment, the biogas plant includes two gas pipelines, one of which serves for the delivery of raw biogas from the fermenter to the biogas treatment plant and the other serves for the delivery of raw biogas from the fermenter to the thermal engine. The two pipelines may be entirely separated or have a common origin that later divides into two separate pipelines. The provision of two pipelines allows that delivery of raw biogas to the gas separation unit and/or to the thermal engine can occur in dependence of the occupancy rate of the respective consumption unit (consumption unit refers in this respect to the thermal engine and the gas separation unit). For instance, all raw biogas from the fermenter can be fed to only one of the consumption units. Alternatively, the raw biogas may also be divided into equal or unequal parts between the two consumption units. The amount of raw biogas delivered to a certain consumption unit is preferably regulated by means of valves, whose through-put is regulated with the aid of a control unit in dependence of the energy requirement of the pertaining consumption unit at the certain point in time.

In view of a particularly energy-efficient operation of the biogas plant, at least part of the thermal energy that is generated during compression of the raw biogas and/or by the thermal engine is preferably used for operating the fermenter. As a result, energy that would otherwise be necessary for heating the fermenter can be saved. The thermal energy generated from the compression unit is preferably distributed within the fermenter. If the thermal energy from the gas compression and the thermal engine is not sufficient for the fermenter's operation, additional external thermal energy may be provided to the fermenter. On the other hand, should more thermal energy be produced than required by the fermenter, the extra energy is preferably fed into a heat storage or a thermal energy grid or is used otherwise within or nearby the biogas plant.

In a further aspect the present invention also relates to a method for the production of biomethane from raw biogas, whereby the method comprises at least the following steps:
Firstly, raw biogas produced by a fermenter is compressed with the aid of a compression unit. The compressed raw biogas is then separated with the aid of a gas separation unit into a product gas, which is enriched in biomethane with respect to the composition of the raw biogas, and into a residual gas that is enriched in carbon dioxide with respect to the composition of the raw biogas. At least a fraction of the residual gas is delivered as part of a combustion gas to a combustion unit and/or as part of an exhaust gas to an exhaust gas treatment unit, which are both part of a thermal engine. In accordance with the present invention, the combustion of the combustion gas within the combustion unit and/or treatment of the exhaust gas in the exhaust gas treatment unit produces energy and at least part of said energy is used for operation of the compression unit.

Preferably, the thermal engine comprises at least one heat exchanger for utilizing the thermal energy that is produced by the combustion unit and/or by the exhaust gas treatment unit.

This reutilization of energy in accordance with the inventive operation method allows that the amount of external energy (e.g. electricity or gas from outside of the biogas plant) used for the operation of the biogas plant can be reduced, preferably to zero. The production of biomethane takes preferably place within a biogas plant as presented above. Thus, the separation of the raw biogas takes preferably place within a gas separation unit that comprises a membrane separation plant and/or a pressure swing adsorption plant. As described above in connection with the preferred embodiments of the inventive biogas plant, gas separation with the aid of a membrane separation unit or a PSA-unit is more energy-efficient and cost saving compared to other known gas separation methods.

In view of an improved energy balance, some energy produced by the thermal engine (electricity or thermal energy) and/or thermal energy generated in the compression unit may also be used for the operation of the fermenter.

It is preferred that the combustion unit receives a variable amount of raw biogas from the fermenter and/or of product gas from the separation unit, depending on the methane concentration of the residual gas. In this case, energy can be produced within the thermal engine without utilizing externally produced energy or fuel (e.g. natural gas) and this allows to use the inventive method in locations that are not connected to a common electricity or gas grid.

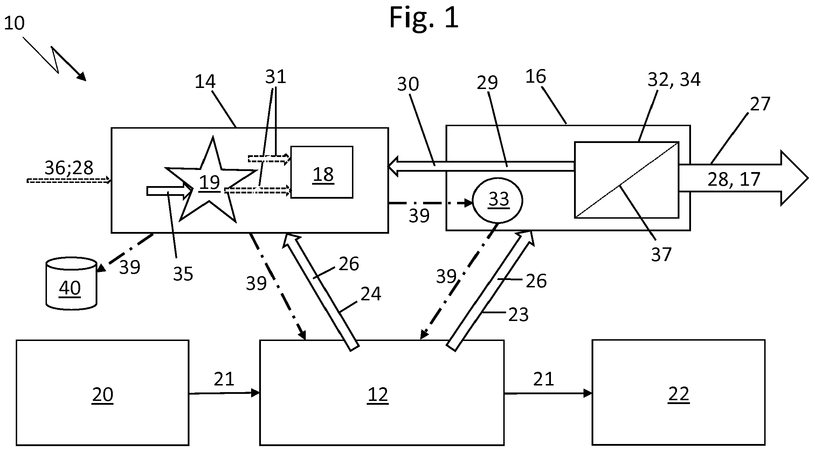

In the following the present invention will be further explained with reference to the embodiment presented in Fig 1, which shows
- Fig. 1: a schematic representation of a biogas plant of the present invention.

An embodiment of a biogas plant 10 in accordance with the present invention as shown in Fig. 1 includes a fermenter 12, a thermal engine 14 and a biogas treatment plant 16 for the production of biomethane 17. The thermal engine 14 includes an exhaust gas treatment unit 18 that is provided with an exhaust gas 31 and a combustion unit 19 that is provided with a combustion gas 35. The term "combustion unit" thereby generally refers to a combustion engine, e.g. a gas (piston) engine, a micro gas turbine or a pilot injection engine, which uses a combustion gas - e.g. natural gas, biogas, or hydrogen - instead of or in addition to a liquid fuel for the conversion of chemical energy into mechanical and/or thermal energy. The thermal engine generally also includes at least one heat exchanger (not shown).

In case of the biogas plant 10 of Fig. 1, a substrate preparation unit 20 provides the fermenter 12 with organic material (biomass) 21. The biomass 21 is fermented in the fermenter 12 and subsequently transported to a substrate postprocessing unit 22.

During the fermentation of the biomass 21 within the fermenter 12, raw biogas 26 is produced. In the shown embodiment, raw biogas 26 from the fermenter 12 is delivered to the biogas treatment plant 16 through a first gas pipeline 23. A second gas pipeline 24 is provided for the delivery of raw biogas 26 from the fermenter 12 to the thermal engine 14. The two pipelines 23, 24 may be entirely separated or have a common origin that later divides into two separate pipelines. The fraction of raw biogas 26 fed to the thermal engine 14 is generally smaller than the fraction that is fed to the biogas treatment plant 16.

Within the biogas treatment plant 16 the raw biogas 26 is separated into a product gas 28 and a residual gas 30. The separation is performed with the aid of a gas separation unit 32 and a compression unit 33 for compression of the raw biogas 26 to be separated. The gas separation unit 32 comprises a membrane separation plant 34.

The membrane separation plant 34 includes at least one membrane 37, by means of which the raw biogas 26 is separated into a first gas stream 27 comprising the product gas 28 and a second gas stream 29 comprising the residual gas 30. The separation occurs on grounds of different permeabilities of biomethane and carbon dioxide through the membrane. The product gas 28 comprises a higher biomethane concentration with respect to the composition of the raw biogas 26, whereas the residual gas 30 comprises more carbon dioxide than the composition of the raw biogas 26.

The gas separation unit 32 is simplified in its construction, which means that it involves less separation steps and/or a reduced membrane area compared to gas separation units known in the art that are built for minimizing the amount of residual biomethane within the residual gas, i.e. the methane slip. Since the biogas plant 10 according to the present invention allows for using the residual biomethane within the residual gas 30 for energy production (see next paragraph), the gas separation unit 32 of the inventive biogas plant can be significantly simplified.

At least a fraction, preferably all, of the residual gas 30 from the separation unit 32 is fed to the thermal engine 14. A fraction between 0% and 100% of said residual gas 30 fed to the thermal engine 14 forms part of the combustion gas 35 that is delivered to the combustion unit 19. In the embodiment presented in Fig. 1, the remaining fraction of the said residual gas 30 fed to the thermal engine 14 is mixed with exhaust gases from the combustion unit 19 and fed as exhaust gas 31 to the exhaust gas treatment unit 18. Said exhaust gas treatment unit 18 is a catalytic gas processing unit and serves for cleaning residual gas 30 that is not fed to the combustion unit 19. In the shown embodiment, exhaust gases from the combustion unit 19 are also fed to the exhaust gas treatment unit 18 as part of the exhaust gas 31, such that the latter can be cleaned, in particular from acidic substances, nitrogen oxides, unburnt hydrocarbons and carbon monoxides, before releasing the cleaned gas into the atmosphere.

By combustion of the combustion gas 35 within the combustion unit 19 and/or treatment of the exhaust gas 31 within the exhaust gas treatment unit 18, mechanical and/or thermal energy 39 is produced. Said energy 39 is used for the operation of the biogas treatment plant 16, specifically the compression unit 33. It may additionally be used for other purposes, e.g. for operation of the fermenter 12 it may be stored in a heat storage 40. If the content of combustible material (in particular biomethane) in the residual gas 30 is too low to allow adequate energy production within the thermal engine 14, additional other combustibles - e.g. raw biogas 26 from the fermenter 12 or natural gas 36 from an external source (e.g. from a public gas grid (not shown)) or biomethane 17 from the biogas treatment plant can be added to the combustion gas 35 or to the exhaust gas 31 of the combustion unit 19. IN addition, at least part of thermal energy 39 produced by the thermal engine 14 and/or the compression unit 33 can also be used for heating the fermenter 12.

## Claims

1. Biogas plant (10) including
a fermenter (12) for the production of raw biogas (26) from biomass (21),
a thermal engine (14) for the production of mechanical and/or thermal energy (39), and
a biogas treatment plant (16) including a compression unit (33) and a gas separation unit (32) for the separation of the raw biogas (26) into a first and second gas stream (27, 29),
wherein the first gas stream (27) consists of a product gas (28) that is enriched in biomethane (17) with respect to the composition of the raw biogas (26) and the second gas stream (29) consists of a residual gas (30) that is enriched in carbon dioxide with respect to the composition of the raw biogas (26),
**characterised in that** the thermal engine (14) includes a combustion unit (19), which is provided with a combustion gas (35) for combustion, and an exhaust gas treatment unit (18), which is provided with an exhaust gas (31) for treatment,
wherein at least one of the combustion gas (35) and the exhaust gas (31) includes at least a fraction of the residual gas (30),
and wherein at least part of the energy (39) produced by combustion of the combustion gas (35) in the combustion unit (19) and/or by treatment of the exhaust gas (31) in the exhaust gas treatment unit (18) is used for operating the compression unit (33).

2. Biogas plant according to claim 1, **characterised in that** the combustion gas (35) includes a variable amount of natural gas (36) and/or raw biogas (26) of the fermenter (12) and/or product gas (28).

3. Biogas plant according to one of claims 1 or 2, **characterised in that** the exhaust gas (31) includes exhaust gases from the combustion unit (19).

4. Biogas plant according to one of claims 1 to 3, **characterised in that** at least one of the combustion gas (35) and the exhaust gas (31) consists of gases from the biogas plant (10).

5. Biogas plant according to one of claims 1 to 4, **characterised in that** the exhaust gas treatment unit (18) is a catalytic gas processing unit.

6. Biogas plant according to one of claims 1 to 5, **characterised in that** the thermal engine (14), in particular the combustion unit (19), and the compression unit (33) are mechanically connected via a rigid coupling.

7. Biogas plant according to one of claims 1 to 5, **characterised in that** the combustion unit (19) and the compression unit (33) are mechanically connected via a variable coupling that allows independent operation of the thermal engine (14) and the compression unit (33).

8. Biogas plant according to one of claims 1 to 7, **characterised in that** the gas separation unit (32) includes a simplified membrane separation plant (34), preferably with two separation steps at most, preferably with only one separation step.

9. Biogas plant according to one of claims 1 to 8, **characterised in that** the gas separation unit (32) has a methane slip of at least 0.25%, preferably at least 1.5%, more preferably at least 3%.

10. Biogas plant according to one of claims 1 to 9, **characterised in that** the combustion gas (35) includes a biomethane concentration of at least 10%, preferably at least 20%, more preferably at least 30%.

11. Biogas plant according to one of claims 1 to 10, **characterised in that** all residual gas (30) from the gas separation unit (32) is delivered directly or indirectly to the thermal engine (14), preferably without prior treatment.

12. Biogas plant according to one of claims 1 to 11, **characterised by** a first gas pipeline (23) for the delivery of raw biogas (26) from the fermenter (12) to the biogas treatment plant (16) and a second gas pipeline (24) for the delivery of raw biogas (26) from the fermenter (12) to the thermal engine (14).

13. Biogas plant according to one of claims 1 to 12, **characterised in that** thermal energy (39) produced during the compression of the raw biogas (26) and/or by the thermal engine (14) is used for operating the fermenter (12).

14. Method for obtaining biomethane from raw biogas, in which
a) raw biogas (26) from a fermenter (12) is compressed with the aid of a compression unit (33);
b) the compressed raw biogas (26) is separated with the aid of a gas separation unit (32) into a product gas (28) that is enriched in biomethane (17) in relation to the raw biogas (26) and into a residual gas (30) that is enriched in carbon dioxide in relation to the raw biogas (26).
c) at least a fraction of the residual gas (30) is delivered as part of a combustion gas (35) to a combustion unit (19) and/or as part of an exhaust gas (31) to an exhaust gas treatment unit (18), which are part of a thermal engine (14) and
d) combustion of the combustion gas (35) in the combustion unit (19) and/or treatment of the exhaust gas (31) in the exhaust gas treatment unit (18) produces energy (39) that is at least partly used for operating the compression unit (33).

15. Method according to claim 14, **characterized in that** the gas separation unit (32) and the thermal engine (14) are part of a biogas plant with the features of one of claims 1 to 13.

16. Method according to claim 14 or 15, **characterized in that** at least part of the energy (39) produced within the thermal engine (14) and/or the compression unit (33) is used for operating the fermenter (12).
